# FASCICULE DE BREVET EUROPEEN

(11) **EP 0 684 307 B1**
(45) Date de publication et mention de la délivrance du brevet: **15.12.1999**
(21) Numéro de dépôt: 94810305.6
(22) Date de dépôt: 27.05.1994
(51) Int. Cl.: C12N 1/20, C12N 1/18

(54) **Procédé de préparation de milieux de culture utilisables pour la culture individuelle de levures et de bactéries lactiques ou la coculture de levures et de bactéries lactiques.**
Verfahren zur Herstellung von Kulturmedien, welche für die individuelle Kultur von Hefen und Milchsäurebakterien oder die Kokultur von Hefen und Milchsäurebakterien verwendbar sind.
Process for the production of culture media usable for the individual culture of yeasts and lactic bacteria or the co-culture of yeasts and lactic bacteria.

(43) Date de publication de la demande: 29.11.1995
(73) Titulaire: AGRANO AG, CH-4123 Allschwil (CH)
(72) Inventeur: Ehret, Aloyse, F-68730 Blotzheim (FR)
(74) Mandataire: Rottmann, Maximilian R.

(56) Documents cités:
- EP-A- 0 093 635
- EP-A- 0 153 057
- EP-A- 0 229 979
- EP-A- 0 295 358
- WO-A-91/04669
- WO-A-92/20777
- BE-A- 816 571
- FR-A- 2 353 235
- GB-A- 372 738
- US-A- 3 868 307
- J.ADRIAN, G.LEGRAND, R. FRANGNE 'Dictionnaire de biochimie alimentaire et de nutrition', 1981, TECHNIQUE ET DOCUMENTATION, PARIS * page 79 *

## Description

L'invention a trait à un procédé de préparation d'un milieu de culture utilisable pour la culture individuelle de levures et de bactéries lactiques ou la coculture de levures et de bactéries lactiques, tel que défini dans les revendications 1 à 4.

Habituellement, la levure de panification est obtenue à partir d'un milieu de culture constitué soit de mélasses sucrières et d'additifs chimiques, soit de céréales. Cependant, ces milieux de culture nécessitaient après la croissance une séparation entre milieu et microorganismes, afin d'obtenir une concentration suffisante de microorganismes permettant une panification dans des conditions compatibles avec une production industrielle.

Dans le cas d'une panification dite "pain au levain", la fabrication du levain nécessite des précultures successives et une adaptation longue des microorganismes au milieu céréalier incompatible avec une production industrielle.

De plus, la publication EP-A1-0.093.635 décrit une composition fermentée pour la préparation d'un levain de panification et un procédé permettant la préparation d'un tel levain par culture de souches sur un milieu céréalier. Le milieu utilisé comprend de l'eau, de la farine, du germe de blé, de l'extrait de levure, du chlorure d'ammonium, du dihydrogénate de potassium, de l'antimousse, de l'alpha amylase et de l'amyloglucosidase. Le procédé de production n'envisage cependant pas l'utilisation de protéases. De plus, la réaction amyloglycolytique est effectué à pH 4.5, ce qui nécessite l'ajout d'acide et donc un contrôle du pH. La composition ainsi que le procédé de sa production ne sont donc pas "tout biologique".

Finalement, la publication WO-A1-92/20.777 décrit un procédé visant à produire de l'éthanol à partir de matières premières contenant des sucres fermentescibles ou des constituants pouvant être convertis en sucres fermentescibles. Le dit procédé comprend les étapes suivantes: (a) liquéfaction des matières premières en présence d'une α-amylase afin d'obtenir une liquéfaction de l'amidon (empois); (b) saccharification de l'empois en présence de glucoamylase pour obtenir les sucres fermentescibles; (c) fermentation des sucres par la levure afin d'obtenir de l'éthanol; et (d) séparation de l'éthanol formé, une protéase fongique étant introduite dans l'empois d'amidon pendant la phase de saccharification et/ou à l'amidon hydrolysé durant la fermentation.

Le but du procédé de cette référence n'est donc pas de produire un milieu de culture qui peut être utilisé pour la culture individuelle de levure et de bactéries lactiques ainsi que pour la coculture de levure et de bactéries lactiques mais la production d'éthanol.

Or, le but de l'invention est de remédier aux inconvénients cités ci-dessus en créant des milieux de culture qui sont utilisables pour la culture de levures et de bactéries lactiques ainsi que pour la coculture de levures et de bactéries lactiques, milieux qui sont exempts de tout additif chimique, et qui permettent l'utilisation directe des microorganismes cultivés pour la panification industrielle.

Ce but est atteint par les mesures définies dans la partie caractérisante de la revendication 1.

Préférablement, le mélange dilué comprend également du sel de table, notamment du sel marin.

Le procédé selon l'invention, suivant la composition du mélange dilué, permet d'obtenir deux milieux à base de céréales, l'un dit "milieu de base", l'autre dit "milieu de culture".

Préférablement, les enzymes protéolytiques comprennent au moins deux protéases différentes, l'une d'elles étant une endoprotéase, et une autre étant une exoprotéase.

Les hydrolyses se font préférablement dans un bioréacteur sans régulation de pH.

Les milieux de culture obtenus selon l'invention permettent notamment la culture de levures, en particulier celles de type *Saccharomyces cerevisiae*, préférablement isolées d'un levain naturel, et particulièrement de la souche *Saccharomyces cerevisiae steineri* DSM 9211.

De même, les milieux de culture obtenus selon l'invention permettent notamment la culture mixte et/ou la culture séquentielle de levures avec des bactéries lactiques, en particulier de levures de type *Saccharomyces cerevisiae*, préférablement isolées d'un levain naturel, et des bactéries lactiques des genres *Lactobacillus*, *Pediococcus* et/ou *Leuconostoc*, préférablement également isolées d'un levain naturel. Ils permettent tout particulièrement la culture de la souche *Saccharomyces cerevisiae steineri* DSM 9211 en culture mixte et/ou culture séquentielle avec une ou plusieurs des souches *Lactobacillus plantarum* DSM 9208, *Lactobacillus brevis* DSM 9209, *Pediococcus pentosaceus* DSM 9210 et *Leuconostoc mesenteroides* DSM 9207.

Dans ce cas, on fait croître dans un bioréacteur dans un système mixte et/ou séquentiel la souche de levure en culture discontinue alimentée avec une ou plusieurs souches bactériennes suivant la nature du produit de panification désirant être obtenu en final.

Ceci permet un contrôle du métabolisme de chaque microorganisme, permettant d'agir sur la concentration des métabolites terminaux, en particulier l'éthanol, l'acide lactique et l'acide acétique. Le choix des microorganismes fondé sur les particularités métaboliques, en particulier les voies métaboliques homofermentaires ou hétérofermentaires, et notamment la régulation de l'activité enzymatique pyruvate oxydase oxygène dépendante (voir: Frey, Le Lait 1992) permet de contrôler d'une manière fine la concentration de l'acide acétique dans le mélange final.

Dans la procédure de coculture, une partie des métabolites terminaux produits par une des espèces microbiennes est réutilisée par les autres espèces influençant les qualités organoleptiques finales (l'acide lactique réutilisé et production de l'acide acétique).

Le système séquentiel permet de différer dans le temps la mise en oeuvre d'un microorganisme par rapport à un autre.

Plusieurs possibilités sont offertes pour la culture mixte et/ou séquentielle:
(1) La mise en culture simultanée d'une ou plusieurs levures et d'une ou plusieurs bactéries lactiques (culture mixte).
(2) La mise en culture d'une ou plusieurs levures pendant une période définie, puis mise en culture dans la culture de levure ou de levures d'une ou plusieurs bactéries lactiques (culture séquentielle).

La mise en culture des bactéries lactiques peut elle-même se faire simultanément (plusieurs souches ensemencées en même temps) ou de façon différée (une première souche mise en coculture avec la levure ou les levures au temps t₁, puis une deuxième souche mise en culture au temps t₂...tₓ).

De la même façon, la culture des bactéries lactiques peut précéder la mise en coculture de la levure ou des levures.

Le Tableau résume ces possibilités sous forme d'un schéma simplifié.

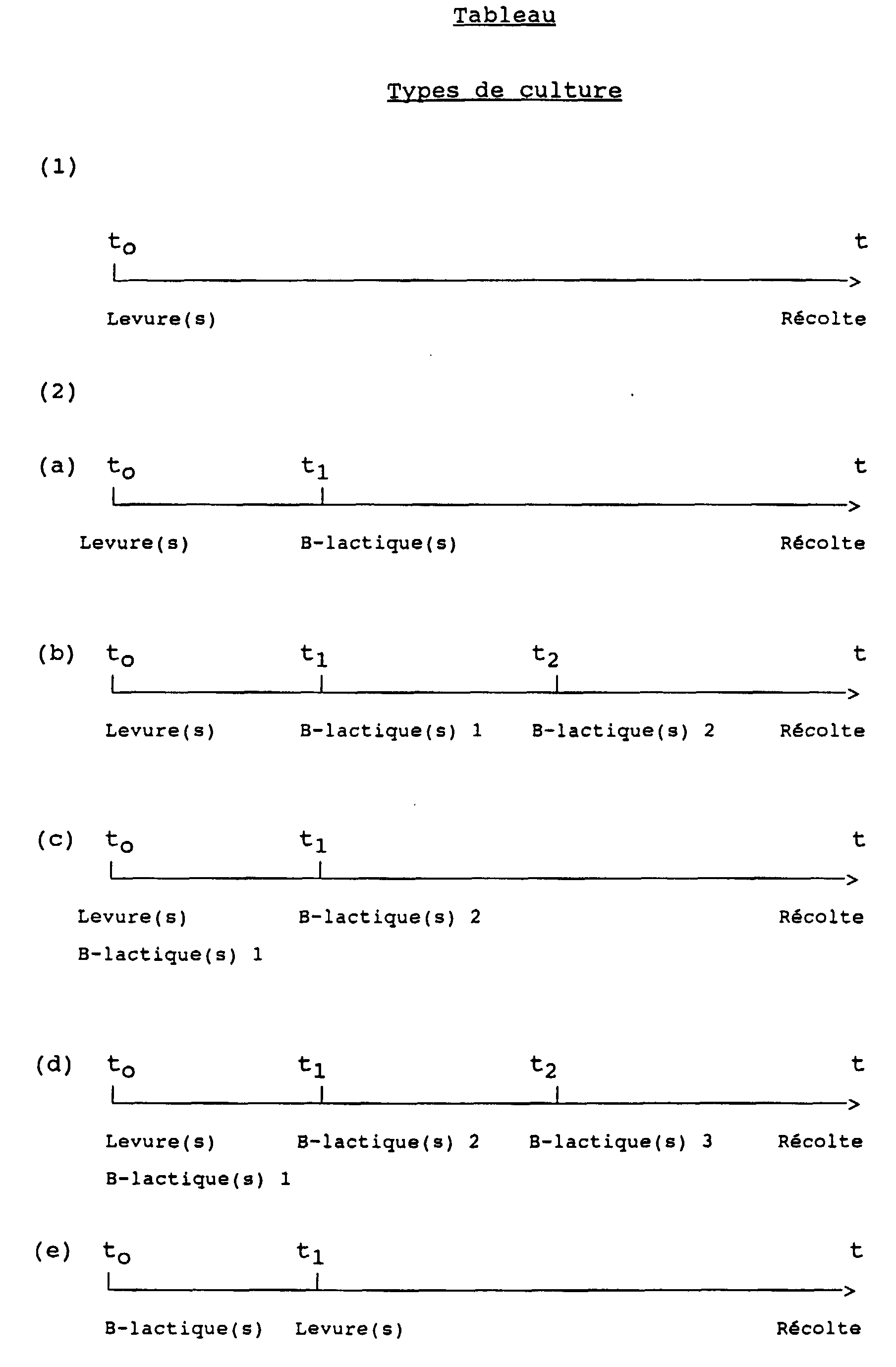

Dans tous les cas, l'utilisation des dits microorganismes, cultivés sur les milieux de culture selon l'invention, permet l'obtention en panification industrielle de produits aux qualités organoleptiques caractéristiques.

### Exemple 1

### Ingrédients utilisés

Les ingrédients suivants sont utilisés pour la préparation des milieux de culture décrits ci-après dans les exemples 2 à 4:
- Grains de blé, moulus extemporanément avant utilisation pour garder l'intégralité des éléments nutritifs. Une analyse type du produit est la suivante:
   - eau environ 13 %,
   - protéines totales environ 12 %,
   - hydrates de carbone environ 69 %,
   - lipides totaux environ 2 %,
   - amidon environ 59 %,
   - cendres 1,5 %.
- Germes de blé, moulus à vitesse réduite avec échauffement réduit contrôlé. Un taux de lipides entre 11 et 12 % et un taux d'amidon inférieur à 10 % sont les critères retenus pour confirmer la qualité du produit. Une analyse type du produit est la suivante:
   - eau environ 13 %,
   - protéines totales environ 31,5 %,
   - hydrates de carbone environ 25 %,
   - lipides totaux environ 11 %,
   - amidon environ 8 %,
   - cendres 5 %.
- Autolysat de levure de qualité alimentaire apportant des vitamines et des acides aminés au milieu. Une analyse type du produit est la suivante:
   - eau environ 3,5 %,
   - protéines totales environ 50,5 %,
   - hydrates de carbone environ 32 %,
   - lipides totaux environ 5 %,
   - amidon environ 1,5 %,
   - cendres 7,5 %.
- Sel marin
- Eau industrielle.

### Exemple 2

### Préparation du milieu dit "milieu de dosage"

Dans un bioréacteur de 15 litres, on mélange 8 litres d'eau, 1660 g de grains de blé moulus, 1000 g de germe de blé, 100 g d'autolysat de levure, 30 g de sel de mer, 1 ml d'une solution d'alpha-amylase (16 unités RAU/g d'amidon à hydrolyser).

Le mélange est porté à 85 °C pendant 20 minutes puis refroidit à 75 °C. Ensuite 2 ml du même enzyme sont ajoutés. La température est maintenue pendant 20 minutes.

Le mélange est refroidit à 60 °C. On ajoute une solution d'amyloglucosidase à raison de 50 ml. L'action de l'enzyme est poursuivie pendant 90 minutes. Le mélange est refroidi à 50 °C et soumis à l'hydrolyse par deux protéases spécifiques, la première étant de la papaïne purifiée et fractionnée, et la deuxième étant de la pancréatine fractionnée.

On utilise 1,5 ml de la première protéase par kg de farine et 2,3 g de la deuxième protéase par kg de farine. L'action des protéases dure 220 minutes.

Le milieu de culture obtenu est stérilisé à 120 °C pendant 20 minutes. Ce milieu parfaitement stable est conservé à +4 °C.

A aucun moment dans la préparation du milieu n'entrent en jeu des additifs chimiques. Le pH final du milieu est voisin de 6,0, par exemple 5,5 à 6,5.

L'ajout d'alpha-amylase en deux temps dans le milieu dit "milieu de dosage" permet d'éviter la gélatinisation irréversible de l'amidon au moment du chauffage au-dessus de 65 °C.

### Exemple 3

### Préparation du milieu dit "milieu de base"

Ce milieu sert d'ensemencement dit en terme technique "de pied de cuve" dans le système de production de la biomasse suivant le procédé de culture discontinue alimentée ("en fed-batch").

Dans un bioréacteur on mélange 10 litres d'eau, 500 g de germe de blé, 70 g d'autolysat de levure et 30 g de sel de marin.

Le milieu est soumis à l'hydrolyse par l'alpha-amylase (2 ml/kg de germe de blé) pendant 20 minutes à 75 °C puis à l'action de deux protéases spécifiques mentionnées ci-dessus, soit de la papaïne purifiée et fractionnée, soit de la pancréatine fractionnée, à 50 °C pendant 20 minutes. Ce milieu est conservé à +4 °C.

A aucun moment dans la préparation du milieu n'entrent en jeu des additifs chimiques. Le pH final du milieu est voisin de 6,0, par exemple 5,5 à 6,5.

### Exemple 4

### Analyse des sucres libérés

Pour l'analyse, les sucres libérés sont dosés par chromatographie liquide à hautes performances, et les acides aminés libérés par l'hydrolyse sous l'action des protéases sont dosés par le réactif à la ninhydrine (S. Moore et W. H. Stein, J. Biol. Chem. 176, 367, 1948).

Les valeurs moyennes obtenues sont:
- Milieu de dosage (Exemple 2):
   - glucose environ 86,5 gramme/litre,
   - maltose environ 11 gramme/litre,
   - acides aminés environ 9 gramme/litre.
- Milieu de base (Exemple 3):
   - glucose environ 6 gramme/litre,
   - maltose environ 12,5 gramme/litre,
   - acides aminés environ 6,5 gramme/litre.

### Exemple 5

### Utilisation des milieux de culture pour la culture de levure

Dans un bioréacteur de 15 litres (volume utile 10 litres) on introduit 5 litres d'un milieu de culture de base préparé selon l'exemple 3 ci-dessus.

A ce milieu de base est ajoutée une souche de levure identifiée à l'espèce *Saccharomyces cerevisiae steineri*, déposée à la Collection allemande de microorganismes (DMS) sous le N^{o} 9211. Cette souche est cultivée sur le milieu de dosage préparé selon l'exemple 2 ci-dessus.

La souche est conservée à -80 °C dans le milieu céréalier contenant du glycérol. La souche est réisolée sur le milieu céréalier solide, et elle est entretenue sur le même milieu par repiquages successifs tous les 15 jours.

Une colonie isolée est ensemencée dans le milieu céréalier liquide. Une deuxième culture est faite avec 20 ml de la première dans un Erlenmeyer de 500 ml contenant 200 ml de milieu céréalier. La densité cellulaire obtenue après 16 heures de culture avec agitation est de 3·10⁸ cellules par ml. Une troisième culture à partir de la deuxième est faite dans 600 ml de la culture précédente. La densité cellulaire obtenue après 8 heures à 30 °C est de 2,5·10⁸ cellules par ml. Le glucose est entièrement métabolisé, et la teneur en éthanol mesurée est voisine de 25 gramme/litre.

600 ml de cette culture de levure en phase exponentielle sont ajoutés aux 5 litres du milieu de base. Le mélange est alimenté en continu en milieu de dosage. La température est maintenue à 30 °C, le pH mesuré en permanence n'est pas régulé, et la pO₂ est maintenue supérieure à 30 %.

La vitesse d'alimentation est asservie à deux paramètres essentiels au bon déroulement du procédé:

### (a) Le paramètre concentration en éthanol du milieu de culture:

La levure est un microorganisme capable de se multiplier en aérobiose et/ou en anaérobiose. Le métabolisme aérobie favorise l'accroissement de la biomasse, alors que le métabolisme anaérobie conduit à la production d'éthanol et de dérivés secondaires recherchés pour leurs qualités organoleptiques. Le procédé selon l'invention permet de contrôler les flux métaboliques entre ces deux voies extrêmes, permettant une bonne croissance et par là l'obtention d'une biomasse finale capable d'être utilisée en panification directe dans des conditions de temps dites "industrielles".
La production d'éthanol traduit un flux métabolique dans la voie anaérobie.

Dans le procédé décrit, la concentration en éthanol est systématiquement recherchée et maintenue entre 0,5 et 10 gramme/litre. Si la concentration passe sous le seuil de 0,5 gramme/litre, la vitesse d'alimentation est augmentée en milieu de dosage. L'alimentation est ralentie en cas de dépassement du seuil de 10 gramme/litre.

### (b) L'aération du milieu de culture:

Ce paramètre est déterminé en continu par la mesure de l'oxygène dissout et est régulé de deux façons, à savoir par pilotage de l'entrée d'air dans le bioréacteur et/ou par l'asservissement de la vitesse de rotation de deux bipales de type Rushton (taille ½ du diamètre du bioréacteur) permettant de piloter le transfert d'O₂.

Dans le procédé décrit, la pression partielle d'O₂ est maintenue supérieure à 10 %. La vitesse d'agitation varie de 500 à 1200 rpm, et le débit d'air stérile passe de 0 à 30 litres/minute.

Dans les conditions décrites, la culture est alimentée durant 16 heures. L'alimentation totale est de 4 litres durant cette période. La densité cellulaire de levure atteint 2·10⁹ cellules par ml (densité de départ 2,5·10⁷ cellules par ml).

La culture obtenue est rapidement refroidie à 3 °C et peut ensuite être conservée sans pertes notables de ses qualités durant 21 jours.

### Variante:

Une postfermentation sur farine à raison de 50 à 300 gramme/litre de ferment pendant 6 heures à 20 °C, suivi d'un refroidissement à 3 °C permet une conservation pendant 30 jours et l'obtention d'un produit de panification aux qualités organoleptiques de type "levain".

### Panification:

Les ferments liquides (issus directement du bioréacteur ou après postfermentation) sont utilisés directement à une concentration de 20 % (poids de farine/volume de ferment) pour ensemencer une pâte traditionnelle faite de farine, d'eau et de sel.

### Variante:

Les ferments sont centrifugés. La réduction de la teneur en eau augmente leur stabilité et permet de diminuer sensiblement la concentration dans le procédé de panification. Ainsi une centrifugation à 8500 G permet d'obtenir un ferment à 75 % d'eau et pourra être utilisé à une concentration de 6 à 8 % dans le mélange de panification.

La densité des pains obtenus est identique à celle obtenue avec la levure pressée traditionnelle (cultivée sur mélasse sucrière) et utilisée à une concentration de 2 à 3 % sur la farine.

### Exemples 6 à 8

### Utilisation des milieux de culture pour la culture mixte de levure et de bactéries lactiques

### Remarque préalable

Le but ultime est d'obtenir une préparation constituée d'une culture mixte permettant la fabrication d'un pain comparable au pain dit de levain traditionnel. Cette croissance microbienne mixte sous-tend des interactions nombreuses de type commensalisme, coopérativité et ammensalisme entre les souches. Le procédé décrit ci-après respecte un compromis visant à produire des lactobacilles en quantité suffisante pour fournir aux pains des arômes et une acidité caractéristiques et à respecter une proportion suffisante de levures (agents levants) permettant d'obtenir sans ajout de levure externe un pain alvéolé de bonne densité.

### Exemple 6

La culture débute par une croissance de la levure. Dans un bioréacteur de 15 litres (volume utile de 10 litres), on introduit successivement 5 litres de milieu de base et 600 ml d'une culture de *Saccharomyces cerevisiae*, en Erlenmeyer comme décrit précédemment. Le mélange est alimenté en milieu de dosage, la température étant maintenue à 30 °C. Le pH mesuré en permanence n'est pas régulé. Il est de 6 au départ et passe à 4,0 - 5,0 en fin de culture mixte selon la souche de bactérie lactique utilisée. L'éthanol est maintenu entre 0,5 et 10 gramme/litre, par asservissement de l'alimentation en milieu de dosage. L'agitation se fait par l'intermédiaire de deux bipales de type Rushton (taille des pales: ½ du diamètre du bioréacteur; coefficient de transfert: 600 mmoles O₂/litre; vitesse d'agitation: 500 à 1200 rpm; débit d'air stérile: variable entre 0 et 30 litres/minute. Le débit est conditionné par la pression partielle d'oxygène mesurée en permanence par une sonde pO₂ (Ingold) et maintenue supérieure à 30 %.

L'ensemencement d'une bactérie lactique, à savoir *Lactobacillus plantarum*, se fait en différé après 8 heures et permet d'ajuster les concentrations des divers microorganismes et d'influencer par là les qualités organoleptiques du produit final.

### Exemple 7

Cet exemple représente une coculture dans laquelle, au départ, il y a une culture mixte levure et *Leuconostoc mesenteroides*. Cette culture mixte est poursuivie durant 8 heures. Au bout de 8 heures est ajoutée une préculture de *Lactobacillus plantarum*. Le produit final est obtenu après 18 heures.

### Exemple 8

Cet exemple représente une coculture de même type que l'exemple 7, mais dans cet essai *Leuconostoc mesenteroides* est remplacé par *Lactobacillus brevis* dans la coculture de départ. *Lactobacillus plantarum* est ajouté après 8 heures.

On remarque dans ce cas l'augmentation de production de l'acide lactique par rapport à l'exemple 2 (2,40 gramme/litre contre 1,81 gramme/litre).

Suivant les cas, les densités cellulaires obtenues entre levures et bactéries sont dans un rapport de 3 à 5 10⁹ levures pour 3 à 5^{.}10⁹ bactéries).

La culture terminée est refroidie à 3 °C le plus rapidement possible. Elle peut être stockée pendant 8 jours sans perdre son aptitude de panification. Elle est utilisée dans un dosage à 20 % (poids/volume).

Les goûts de "levain" des pains obtenus sont excellents.

## Revendications

1. Procédé de préparation d'un milieu de culture, ce milieu de culture étant utilisable pour la culture individuelle de levures et de bactéries lactiques ainsi que pour la coculture de levures et de bactéries lactiques et étant exempt de tout additif chimique autre que les ingredients mentionnés ci-après, **caractérisé:**
- en ce qu'on prépare un mélange dilué aqueux comprenant au moins de la farine intégrale et/ou du germe de blé ainsi que de l'autolysat de levure et du sel de table, ce mélange comprenant de l'amidon et du gluten;
- en ce qu'on ajoute au moins une alpha-amylase et au moins une amyloglucosidase pour hydrolyser dans ce mélange l'amidon totalement en sucres fermentescibles;
- en ce qu'on ajoute au moins une enzyme protéolytique en qualité alimentaire pour hydrolyser avec ménagement dans ce mélange au moins une partie du gluten en des peptides aromatiques et des acides aminés libres, ceci en quantité suffisante pour promouvoir lors de l'utilisation du milieu de culture la croissance microbienne;
- les deux hydrolyses se faisant sans régulation de pH; et
- en ce qu'on stérilise le produit obtenu.

2. Procédé selon la revendication 1, **caractérisé** en ce que le sel de table est du sel marin.

3. Procédé selon la revendication 1 ou 2, **caractérisé** en ce que les enzymes protéolytiques comprennent au moins deux protéases différentes, l'une d'elles étant une endoprotéase, et une autre étant une exoprotéase.

4. Procédé selon une des revendications 1 à 3, **caractérisé** en ce que les hydrolyses se font dans un bioréacteur.

## Claims

1. Method of preparing a culture medium, this culture medium being utilizable for the individual culture of yeasts and lactic acid bacteria as well as for the coculture of yeasts and lactic acid bacteria and being free of any chemical additive other than the ingredients mentioned below, **characterized**:
- in that an aqueous dilute mixture comprising at least wholemeal flour and/or wheat germ as well as yeast autolysate and table salt is prepared, this mixture comprising starch and gluten;
- in that at least one alpha-amylase and at least one amyloglucosidase are added in order to completely hydrolyse the starch to fermentable sugars in this mixture;
- in that at least one food-grade proteolytic enzyme is added in order to hydrolyse, in a controlled manner, at least part of the gluten to aromatic peptides and free amino acids in this mixture, in sufficient quantity to promote microbial growth during the use of the culture medium;
- both hydrolyses being carried out without regulation of pH; and
- in that the product obtained is sterilized.

2. Method according to Claim 1, **characterized** in that the table salt is sea salt.

3. Method according to Claim 1 or 2, **characterized** in that the proteolytic enzymes comprise at least two different proteases, one of them being an endoprotease, and another being an exoprotease.

4. Method according to one of Claims 1 to 3, **characterized** in that the hydrolyses are carried out in a bioreactor.

## Patentansprüche

1. Verfahren zur Herstellung eines Kulturmediums, welches zur individuellen Kultur von Hefen und Milchsäurebakterien sowie zur Kokultur von Hefen und Milchsäurebakterien verwendbar ist und frei ist von jeglichen chemischen Zusätzen ausser den nachstehend genannten, **dadurch gekennzeichnet:**
- dass man eine verdünnten wässerige Mischung herstellt, welche Vollkornmehl und/oder Weizenkeime sowie Hefeautolysat und Kochsalz enthält und welche Stärke und Gluten enthält;
- dass man wenigstens eine alpha-Amylase und wenigstens eine Amyloglucosidase zugibt, um in dieser Mischung die Stärke ganz in vergärbare Zucker zu hydrolysieren;
- dass man wenigstens ein proteolytisches Enzym von Lebensmittelqualität zugibt, um in dieser Mischung kontrolliert wenigstens ein Teil des Glutens in aromatische Peptide und freie Aminosäuren zu hydrolysieren, und zwar in einer Menge, welche ausreicht, um bei der Verwendung des Kulturmediums das Wachstum der Mikroorganismen zu fördern; wobei beide Hydrolysen ohne pH-Regluierung durchgeführt werden; und
- dass man das erhaltene Produkt sterilisiert.

2. Verfahren nach Anspruch 1, **dadurch gekennzeichnet,** dass das Kochsalz Meersalz ist.

3. Verfahren nach Anspruch 1 oder 2, **dadurch gekennzeichnet,** dass die proteolytischen Enzyme aus mindestens zwei verschiedene Proteasen bestehen, von denen die eine eine Endoprotease, die andere eine Exoprotease ist.

4. Verfahren nach einem der Ansprüche 1 bis 3, **dadurch gekennzeichnet,** dass die Hydrolysen in einem Bioreaktor durchgeführt werden.
